(19) Europäisches Patentamt
European Patent Office
Office européen des brevets

(11) **EP 3 527 229 B1**

(12) **EUROPEAN PATENT SPECIFICATION**

(45) Date of publication and mention of the grant of the patent:
**03.07.2024 Bulletin 2024/27**

(21) Application number: **17862277.5**

(22) Date of filing: **13.10.2017**

(51) International Patent Classification (IPC):
***C12N 15/113*** *(2010.01)* ***A61K 31/712*** *(2006.01)*
***A61K 9/127*** *(2006.01)*

(52) Cooperative Patent Classification (CPC):
**C12N 15/1135; A61K 9/127; A61K 9/51; A61K 31/7088; A61K 47/10; A61K 47/24; A61K 47/26; A61K 48/00; A61P 35/00; B82Y 5/00;** C12N 2310/11; C12N 2310/315; C12N 2310/321

(86) International application number:
**PCT/CN2017/106042**

(87) International publication number:
**WO 2018/072646 (26.04.2018 Gazette 2018/17)**

(54) **LIPID NANOPARTICLE OF ANTISENSE OLIGONUCLEIC ACID FOR INHIBITING BCL-2 AND PREPARATION METHOD THEREFOR**

LIPIDNANOPARTIKEL AUS ANTISENSE-OLIGONUKLEINSÄURE ZUR HEMMUNG VON BCL-2 UND HERSTELLUNGSVERFAHREN DAFÜR

NANOPARTICULE LIPIDIQUE D'ACIDE OLIGONUCLÉIQUE ANTISENS DESTINÉE À INHIBER BCL-2 ET SON PROCÉDÉ DE PRÉPARATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.10.2016 CN 201610903392**

(43) Date of publication of application:
**21.08.2019 Bulletin 2019/34**

(73) Proprietors:
- **Nanjing Luye Pharmaceutical Co., Ltd. Jiangsu 210061 (CN)**
- **Nanjing Asec Nano Biomedicine Co., Ltd. Jiangsu 210061 (CN)**

(72) Inventors:
- **CHENG, Guang Jiangsu 210061 (CN)**
- **CHEN, Wenzhong Jiangsu 210061 (CN)**
- **QIN, Lili Jiangsu 210061 (CN)**

(74) Representative: **Gong, Jinping**
**CocreateIP**
**Neumarkterstr. 21**
**81673 München (DE)**

(56) References cited:
**EP-A1- 1 637 144**
**WO-A1-2006/007712**
**WO-A1-2007/051303**
**WO-A1-2010/057160**
**WO-A2-2008/147438**
**WO-A2-2009/028824**
**WO-A2-2009/120247**
**WO-A2-2012/006380**
**CN-A- 102 215 820**

- **FOLDVARI MARIANNA ET AL: "Non-viral gene therapy: Gains and challenges of non-invasive administration methods", JOURNAL OF CONTROLLED RELEASE, ELSEVIER, AMSTERDAM, NL, vol. 240, 11 December 2015 (2015-12-11), pages 165-190, XP029759403, ISSN: 0168-3659, DOI: 10.1016/J.JCONREL.2015.12.012**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 527 229 B1

- **IMMORDINO MARIA LAURA ET AL: "Stealth liposomes: review of the basic science, rationale, and clinical applications, existing and potential", INTERNATIONAL JOURNAL OF NANOMEDICINE, DOVE MEDICAL PRESS LTD, AUCKLAND, NZ, vol. 1, no. 3, September 2006 (2006-09), pages 297-315, XP009145524, ISSN: 1176-9114**
- **YANG XIAOJUAN ET AL: "Transferrin Receptor-Targeted Lipid Nanoparticles for Delivery of an Antisense Oligodeoxyribonucleotide against Bcl-2", MOLECULAR PHARMACEUTICS, AMERICAN CHEMICAL SOCIETY, US, vol. 6, no. 1, 15 December 2008 (2008-12-15), pages 221-230, XP008163773, ISSN: 1543-8384, DOI: 10.1021/MP800149S [retrieved on 2008-12-15]**
- **Shih-Jiuan Chiu ET AL: "Efficient delivery of an antisense oligodeoxyribonucleotide formulated in folate receptor-targeted liposomes", Anticancer Research, April 2006 (2006-04), pages 1049-1056, XP55052701, Retrieved from the Internet: URL:http://ar.iiarjournals.org/content/26/2A/1049.full.pdf#page=1&view=FitH [retrieved on 2013-02-07]**
- **SU-EON JIN ET AL: "Cellular delivery of cationic lipid nanoparticle-based SMAD3 antisense oligonucleotides for the inhibition of collagen production in keloid fibroblasts", EUROPEAN JOURNAL OF PHARMACEUTICS AND BIOPHARMACEUTICS, vol. 82, no. 1, 15 June 2012 (2012-06-15), pages 19-26, XP55724725, NL ISSN: 0939-6411, DOI: 10.1016/j.ejpb.2012.05.015**
- **NORBERT MAURER ET AL: "Spontaneous Entrapment of Polynucleotides upon Electrostatic Interaction with Ethanol-Destabilized Cationic Liposomes", BIOPHYSICAL JOURNAL, vol. 80, no. 5, May 2001 (2001-05), pages 2310-2326, XP055550920, AMSTERDAM, NL ISSN: 0006-3495, DOI: 10.1016/S0006-3495(01)76202-9**
- **CHENG XINWEI ET AL: "Lipid Nanoparticles Loaded with an Antisense Oligonucleotide Gapmer Against Bcl-2 for Treatment of Lung Cancer", PHARMACEUTICAL RESEARCH, SPRINGER NEW YORK LLC, US, vol. 34, no. 2, 28 November 2016 (2016-11-28), pages 310-320, XP036133835, ISSN: 0724-8741, DOI: 10.1007/S11095-016-2063-5 [retrieved on 2016-11-28]**
- **MEYER, O. et al.: "Cationic Liposomes Coated with Polyethylene Glycol As Carriers for Oligonucleotides", The Journal of Biological Chemistry, vol. 273, no. 25 , pages 15621-15627, XP000858874, DOI: doi:10.1074/jbc.273.25.15621**
- **CHENG, XINWEI et al.: "The Role of Helper Lipids in Lipid Nanoparticles (LNPs) Designed for Oligonucleotide Delivery", Advanced Drug Delivery Reviews, vol. 99 , pages 129-137, XP029445785, DOI: doi:10.1016/j.addr.2016.01.022**


Remarks:

The complete document including Reference Table(s) and the Sequence Listing(s) can be downloaded from the EPO website

## Description

### Technical Field

The invention relates to the technical field of biology, in particular to a lipid nanoparticle comprising antisense oligonucleotides for inhibiting bcl-2 as specified in any of claims 1-8.

### Background Art

Antisense oligonucleotides generally consist of 18-22 nucleotides and are selectively bound to the target messenger RNA by the principle of complementary base pairing, thereby blocking or inhibiting the function of specific messenger RNAs and regulating the expression of proteins of subsequent target genes.

Bcl-2 is a gene that inhibits apoptosis. This gene can promote cell division, expansion, differentiation, and has higher expression in most tumor cells, thus the tumor can proliferate and metastasize by up-regulating this gene. G3139 is an antisense oligonucleotide consisting of 18 nucleotides having a nucleic acid sequence of 5'-TCT CCC AGC GTG CGC CAT-3', which can be bound by the principle of complementary base pairing to messenger RNA encoding the bcl-2, therefore inhibiting the expression of bcl-2 genes and downstream proteins. WO 2009/120247 A2 describes nanoparticles comprising antisense oligonucleotides targeting Bc1-2 such as G3139.

However, according to previous reports, most antisense oligonucleotides lack a suitable drug delivery system and are difficult to enter cells on their own. They also have a weak binding effect on target messenger RNA and are difficult to maintain the inhibitory binding effect for a long time, and most antisense oligonucleotides are readily degraded under the action of nucleases in the plasma and lose their therapeutic effect. Although G3139 has a certain therapeutic effect after many years of clinical trials, it has finally failed to obtain approval because its therapeutic effect cannot reach the US FDA standard.

There is no prior art where lipid nanoparticle membrane materials include a combination between a Tween (®) compounds and a long-chain polyethylene glycol (PEG) derivative such as TPGS. Tween has been used alone. It has short PEG chains, when can increase the repellency among the nanoparticles to prevent their aggregation and destabilization. On the other hand, due to the lack of long-chain PEG embedded on the surface of nanoparticles, such nanopreparations are easily phagocytosed by phagocytes and lose their function. This is also because Tween is easily lost in the systemic circulation.

Another example is the use of polyethylene glycol (PEG) derivatives alone in nanoparticles, such as TPGS. Although TPGS has a longer PEG chain, it can prevent the recognition by the phagocytic system to a certain extent, and increases systemic circulation time. However, if there are a lot of TPGS, the nanoparticles will be difficult to be effectively taken up by the target tumor cells because of the steric hindrance effect.

For G3139, an antisense oligonucleotide, most of the previous modifications were phosphorothioated (PS) substitutions. Although these modifications can improve the stability of the antisense oligonucleotides to a certain degree, its binding affinity with the messenger RNA was reduced.

### Summary

1. Technical problems to be solved by the invention

The purpose of the present invention is to overcome the above technical deficiencies and provide a composition for improving the stability of the nanoparticle itself, thereby promoting the release of the drug in the tumor tissue and reducing the possibility of being degraded, and at the same time improving the resistance of the antisense oligonucleotides to the nucleases and improving the matching accuracy and binding capacity with the messenger RNA.

2. Technical solutions

To achieve the above purpose, the technical solution provided by the present invention is as follows:

a lipid nanoparticle comprising antisense oligonucleotides for inhibiting bcl-2 prepared by coating an antisense oligonucleotide with a membrane material, and the nucleic acid sequence is 5'-TCT CCC AGC GTG CGC CAT-3', or 5' UCU CCC AGC GTG CGC CAU 3'; and

the membrane material comprises a cationic lipid, a neutral phospholipid, cholesterol, Tween, a polyethylene glycol derivative in a molar ratio of (25-35):(40-50):(15-25):(1-5):(1-5);

wherein the PEG in the polyethylene glycol derivative includes monomethoxy polyethylene glycol having a molecular weight of from 550 to 5,000;
; and

the Tween is Tween-80.

In a further technical solution, the cationic lipid includes: DOTAP, DOTMA, DDAB, and DODMA;

the neutral phospholipid includes: egg PC, DOPC, DSPC, DPPC, and DMPC;

the polyethylene glycol derivative includes: mPEG-DPPE, mPEG-DMPE, mPEG-DSPE, TPGS, and mPEG-cholesterol.

In a further technical solution, the PEG molecular weights of the polyethylene glycol derivative are 550, 750, 1,000, 2,000, 3,000, and 5,000.
In a further technical solution, the polyethylene glycol (PEG) derivative is a substance formed by linking a hydrophilic polyethylene glycol (PEG) chain or a methylated polyethylene glycol chain (mPEG) to a hydrophobic structure that can be embedded into a phospholipid bilayer; and the hydrophobic structure includes cholesterol, vitamin E, dipalmitoylphosphatidylethanolamine (DPPE), dimyristoylphosphatidylethanolamine (DMPE), dimyristoylglycerol (DMG), or structural analogs.
In a further technical solution, a modification to the antisense oligonucleotide 5' UCU CCC AGC GTG CGC CAU 3' is a 2'-O-Me modification to the first three nucleotides at both ends thereof, and a phosphorothioate modification to the entire chain; or phosphorothioate modification to the entire chain of antisense oligonucleotide 5'-TCT CCC AGC GTG CGC CAT-3'.
In a further technical solution, the membrane material includes DOTAP, Egg PC, cholesterol, Tween 80, and TPGS in a molar ratio of 25:45:20:5:5.
In a further technical solution, the membrane material includes DOTMA, Egg PC, Cholesterol, Tween 80, and TPGS in a molar ratio of 30:45:20:5:5.
In a further technical solution, the membrane material includes DOTAP, DSPC, cholesterol, Tween 80, and TPGS in a molar ratio of 30:50:20:5:5.
In a further technical solution, the membrane material includes DOTAP, Egg PC, cholesterol, Tween 80, and mPEG 2000-DPPE in a molar ratio of 30:45:20:5:5.

DOTAP: 1,2-dioleoyloxy-3-trimethylaminopropyl chloride

DOTMA: 1,2-dioleyloxy-3-trimethylaminopropyl chloride

DDAB: Dioctadecyldimethylammonium bromide

Egg PC: egg yolk phosphatidylcholine

DOPC: dioleoyl phosphatidylcholine

DSPC: distearoyl phosphatidylcholine

DPPC: dipalmitoylphosphatidylcholine

DODMA: 1,2-dioxyoctadecene-3-dimethylaminopropane

DMPC: dimyristoylphosphatidylcholine

DPPE: dipalmitoylphosphatidylethanolamine

DMPE: dimyristoylphosphatidylethanolamine

TPGS: succinate

The phosphorothioate modification is to replace an unbridged oxygen atom in the phosphate group by a sulfur

atom. As shown in the figure below, the structure of the entire oligonucleotide chain is less affected, and resistance to various nucleases is improved to a large extent.

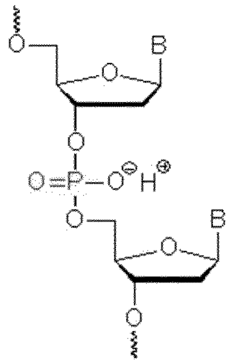

5'-3' Phosphodiester linkage

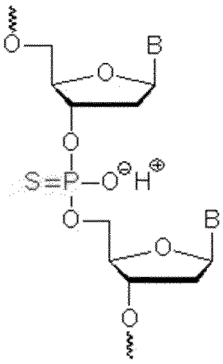

5'-3' Phosphorothioate linkage

3. Beneficial effects

Compared with the prior art, the present invention has the following significant advantages:

1. For the first time, a dual pegylated reagent (Tween and polyethyleneglycol derivative) was used for the nanoparticle membrane material of the present invention, which can improve the stability of the nanoparticle itself to some extent, and promote release and reduce possibility of degradation of the drug prior to reaching the tumor tissue.

Tween as a pegylated reagent, is usually used in the preparation of nano-preparations. The Tween series have short-chained PEG, Tween series such as Tween 20/40/60/80 can improve repulsive interaction among the nanoparticles in nano-preparations, so that the nanoparticles are less prone to aggregation and can improve the stability of nano-preparations to a certain extent, and the particle size growth would be smaller in long-term storage.

The polyethylene glycol derivative such as TPGS can be used as a component of another commonly used nano-preparation, which has a longer PEG chain to promote the circulation of the nanoparticles in the blood and prevent loss due to uptake by reticuloendothelial cells or phagocytes. Recognition of the immune system can be avoided to a certain extent, long circulation time can be achieved, and longer PEG chain will affect the cell uptake to a certain extent.

We found that combination of both, of the longer PEG chain and the shorter Tween, and of these two pegylated reagents by the present invention can effectively improve the stability, long circulation time, target cell release, and gene down regulation efficacy to the target cells of the antisense oligonucleotide-loaded nanoparticles in the plasma.

2. The stability test of the Tween series and the TPGS composition at 4 degrees on the 27th day in the present invention, as shown in FIG. 1, in the one-month time, the change of the particle size of the nano-preparation was smaller, indicating that Tween can maintain the overall stability of the preparation.

The specific experimental data are as follows: in the stability test of Tween 80 at 4 degrees on the 27th day, the nanoparticle size of the nano-preparation was changed from 126 nm to 181 nm, and the stability was most obvious;

in the stability test of Tween 20 at 4 degrees on the 27th day, the nanoparticle size of the nano-preparation was changed from 99.8 nm to 274.2 nm, and the stability was better;

in the stability test of Tween 40 at 4 degrees on the 27th day, the nanoparticle size of the nano-preparation was changed from 138.5 nm to 305.9 nm, and the stability was better;

in the stability test of Tween 60 at 4 degrees on the 27th day, the nanoparticle size of the nano-preparation was changed from 76.7 nm to 218.6 nm, and the stability was better.

3. The membrane material of the lipid nanoparticle of the present invention includes a cationic lipid, a phospholipid, cholesterol, Tween, and a polyethylene glycol derivative which were combined in a certain ratio so that the prepared nano-membrane material can effectively reduce costs, and the stability is good, cell receptivity is high, and it is a good nano-carrier.

4. In the present invention, G3139 was subjected to dual chemical modification of 2'-O-Methyl (2'-O-Me) and phosphorothioate (PS). The first three nucleotides at both ends were subjected to 2'-O-Me modification, which can

improve the stability of G3139 oligonucleotide in plasma to a certain extent, while the entire chain was subjected to phosphorothioate modification. Such RNA/DNA/RNA structure can promote RNase H function to specific target messenger RNA sequences for degradation, and improve its resistance to nucleases and improve the matching accuracy and binding ability with messenger RNA to some extent.

When three nucleotides were subjected to 2'-O-Me modification at both ends, the stability of the G3139 antisense oligonucleotide can be greatly improved without affecting the recognition and degradation to the target gene. However, when two (G3139-GAP-LNPs-2) or four nucleotides (G3139-GAP-LNPs-4) were modified by 2'-O-Me, no such effect was produced. The solution provided by the present invention has advantages of good modification effect, being easy to reach the target site, and fairly good inhibitory effect.

5. The nanoparticles of the present invention have a fairly good inhibitory effect on the growth of tumor cells and specific target genes, and particularly have a fairly good inhibitory effect on KB cervical cancer cells.

6. The high-salinity solution added in the preparation of the present invention can dissociate the free oligonucleotides adsorbed on the surface of the nanoparticles, and reduce the larger particle size generated due to the adsorption of the oligonucleotides, and the free oligonucleotides can be removed in a subsequent step of dialysis.

7. The non-claimed method for preparing the lipid nanoparticle of the present invention is an ethanol stepwise dilution method. However, the method of equivoluminal in-line mixing method used in the prior art is relatively complicated, in which two pumps and different types of liquid storage tanks are required, and the method is not suitable for industrial production. Compared with the prior art, the described method is more conducive to the dynamic balance of the two systems and promotes the stability of the subsequent suspension system from the perspective of mixing kinetics.

## Brief Description of the Drawings

FIG. 1 is a graph showing the stability test of the composition of Tween series and TPGS at 4 degrees for 27 days according to the present invention;
FIG. 2 is a graph showing the regulation of gene level (Bcl-2) G3139 in KB cancer cells;
FIG. 3 is a graph showing the regulation of gene level (Bcl-2) G3139 in A549 lung cancer cells;
FIG. 4 is a graph showing the regulation of gene level (Bcl-2) G3139 in LNCaP prostate cancer cells;
FIG. 5 is a graph showing the tumor inhibition rate;
FIG. 6 is a graph showing the survival test.

Detailed Description

The present invention and its embodiments are described schematically below, the description is non-limiting.

Example 1

A method for preparing a lipid nanoparticle for antisense oligonucleotides inhibiting bcl-2, comprising the following specific steps:

(1) dissolving DOTAP, Egg PC, cholesterol, Tween 80, and TPGS in a molar ratio of 25:45:20:5:5 in 80% ethanol to obtain an ethanol solution. Dissolve the antisense oligonucleotide 5'-TCT CCC AGC GTG CGC CAT-3' in a PBS buffer (1X pH=7) to obtain an antisense oligonucleotide solution;
(2) mixing the resulting mixed ethanol solution and the antisense oligonucleotide solution in equal volumes to obtain a 40% final ethanol concentration mixed solution;
(3) further diluting the 40% final ethanol concentration mixed solution obtained in step (2) with the PBS solution in equal volume; repeatedly diluting the final ethanol concentration mixed solution with the PBS solution (1X pH=7.4) in equal volume until a preparation mixed solution with a final ethanol concentration of less than 5% is obtained;
(4) adding a high-salinity solution to the preparation mixed solution obtained in step (3) to obtain a 0.1 M mixed solution to dissociate the free oligonucleotide bound to the surface of the nanoparticles.
(5) removing ethanol and free antisense oligonucleotides from the mixed solution obtained in step (4) by an ultrafiltration device having a molecular weight cutoff of 10,000 daltons.
(6) filtering and sterilizing a product obtained in step (5) through a filter membrane or a filter element with a pore size of 0.22 μm to obtain the lipid nanoparticle.

The method for preparing a lipid nanoparticle comprising antisense oligonucleotides for inhibiting bcl-2 is not claimed by the present invention.

Example 2

A method for preparing a lipid nanoparticle comprising antisense oligonucleotides for inhibiting bcl-2, comprising the following specific steps:

(1) dissolving DOTMA, DOPC, Cholesterol, Tween 40, and mPEG2000-DPPE in a molar ratio of 35:40:15:1:1 in 80% ethanol to obtain an ethanol solution. Dissolve the antisense oligonucleotide 5'UCU CCC AGC GTG CGC CAU 3' with phosphorothioate modification on the entire chain, modifying both ends by 2'-O-Me and dissolving in a PBS buffer (1XpH=7) to obtain an antisense oligonucleotide solution;

(2) mixing the resulting mixed ethanol solution and the antisense oligonucleotide solution in equal volume to obtain a 40% final ethanol concentration mixed solution;

(3) further diluting the 40% final ethanol concentration mixed solution obtained in step (2) with the PBS solution in equal volume; repeatedly diluting the final ethanol concentration mixed solution with the PBS solution (1X pH=7.4) in equal volume until a preparation mixed solution with a final ethanol concentration of less than 5% is obtained;

(4) adding a high-salinity solution to the preparation mixed solution obtained in step (3) to obtain a mixed solution having a final concentration of 0.3 M and containing high-concentration salt;

(5) removing ethanol and free antisense oligonucleotides from the mixed solution obtained in step (4) by an ultrafiltration device having a molecular weight cutoff of 50,000 daltons;

(6) filtering and sterilizing a product obtained in step (5) through a filter membrane or a filter element with a pore size of 0.2 μm to obtain the lipid nanoparticle.

The method for preparing a lipid nanoparticle for antisense oligonucleotides inhibiting bcl-2 is not claimed by the present invention.

Example 3

A method for preparing a lipid nanoparticle comprising antisense oligonucleotides for inhibiting bcl-2, comprising the following specific steps:

(1) dissolving DDAB, DSPC, cholesterol, Tween 60, and mPEG2000-DPPE in a molar ratio of 30:50:25:3:3 in 80% ethanol to obtain an ethanol solution. Dissolve the antisense oligonucleotide 5'UCU CCC AGC GTG CGC CAU 3' with phosphorothioate modification on the entire chain, and modifications on both ends by 2'-O-Me, in a PBS buffer (1X pH=7) to obtain an antisense oligonucleotide solution;

(2) mixing the resulting mixed ethanol solution and the antisense oligonucleotide solution in equal volume to obtain a 40% final ethanol concentration mixed solution;

(3) further diluting the 40% final ethanol concentration mixed solution obtained in step (2) with the PBS solution in equal volume; repeatedly diluting the final ethanol concentration mixed solution with the PBS solution (1X pH=7.4) in equal volume until a preparation mixed solution with a final ethanol concentration of less than 5% is obtained;

(4) adding a high-salinity solution to the preparation mixed solution obtained in step (3) to obtain a mixed solution having a final concentration of 1 M and containing high-concentration salt;

(5) removing ethanol and free antisense oligonucleotides from the mixed solution obtained in step (4) by an ultrafiltration device having a molecular weight cutoff of 100,000 daltons;

(6) filtering and sterilizing a product obtained in step (5) through a filter membrane or a filter element with a pore size of 0.22 μm to obtain the lipid nanoparticle.

The method for preparing a lipid nanoparticle for antisense oligonucleotides inhibiting bcl-2 is not claimed by the present invention.

Example 4

(1) DOTAP/Egg PC/Cholesterol/Tween 80/TPGS=25:50:20:5:5
(2) DOTAP/Egg PC/Cholesterol/Tween 80/TPGS=30:50:20:5:5
(3) DOTAP/Egg PC/Cholesterol/Tween 80/TPGS=35:50:20:5:5

| Preparation | Particle size (nm) | Potential (mV) | Encapsulation rate (%) |
|---|---|---|---|
| 1 | 123.4 | 7.45 | 89.2% |
| 2 | 132.5 | 7.96 | 83.8% |
| 3 | 128.1 | 8.55 | 84.9% |

A single-factor experiment was performed by adjusting the molar ratio of DOTAP-positive phospholipids, we found that under the condition that other components were always the same, the particle size and encapsulation rate of the first group were significantly superior than those of the other two groups, the potential remained substantially the same, and therefore we selected the first group as the optimal prescription for the following screening.
(1) DOTAP/Egg PC/Cholesterol/Tween 80/TPGS=25:40:20:5:5
(2) DOTAP/Egg PC/Cholesterol/Tween 80/TPGS=25:45:20:5:5
(3) DOTAP/Egg PC/Cholesterol/Tween 80/TPGS=25:50:20:5:5

| Preparation | Particle size (nm) | Potential (mV) | Encapsulation rate (%) |
|---|---|---|---|
| 1 | 138.6 | 7.53 | 83.6% |
| 2 | 123.4 | 7.45 | 89.2% |
| 3 | 136.0 | 7.21 | 84.2% |

A single-factor experiment was performed by adjusting the molar ratio of Egg PC phospholipids, we found that under the condition that other components were always the same, the particle size and encapsulation rate of the second group were significantly superior than those of the other two groups, the potential remained substantially the same, and therefore we selected the second group as the optimal prescription for the following screening.
(1) DOTAP/Egg PC/Cholesterol/Tween 80/TPGS=25:45:15:5:5
(2) DOTAP/Egg PC/Cholesterol/Tween 80/TPGS=25:45:20:5:5
(3) DOTAP/Egg PC/Cholesterol/Tween 80/TPGS=25:45:25:5:5

| Preparation | Particle size (nm) | Potential (mV) | Encapsulation rate (%) |
|---|---|---|---|
| 1 | 120.4 | 7.78 | 80.4% |
| 2 | 123.4 | 7.45 | 89.2% |
| 3 | 143.4 | 7.03 | 86.2% |

A single-factor experiment was performed by adjusting the molar ratio of cholesterol, we found that under the condition that other components were always the same, the particle size and encapsulation rate of the second group were significantly superior than those of the other two groups, the potential remained substantially the same, and therefore we selected the second group as the optimal prescription for the following screening.
(1) DOTAP/Egg PC/Cholesterol/Tween 80/TPGS=25:45:20:1:5
(2) DOTAP/Egg PC/Cholesterol/Tween 80/TPGS=25:45:20:3:5
(3) DOTAP/Egg PC/Cholesterol/Tween 80/TPGS=25:45:20:5:5

| Preparation | Particle size (nm) | Potential (mV) | Encapsulation rate (%) |
|---|---|---|---|
| 1 | 135.4 | 9.23 | 78.4% |
| 2 | 128.0 | 8.53 | 84.7% |

(continued)

| Preparation | Particle size (nm) | Potential (mV) | Encapsulation rate (%) |
|---|---|---|---|
| 3 | 123.4 | 7.45 | 89.2% |

A single-factor experiment was performed by adjusting the molar ratio of Tween 80, we found that under the condition that other components were always the same, the particle size and encapsulation rate of the third group were significantly superior than those of the other two groups, the potential remained substantially the same, and therefore we selected the third group as the optimal prescription for the following screening.
(1) DOTAP/Egg PC/Cholesterol/Tween 80/TPGS=25:45:20:5:1
(2) DOTAP/Egg PC/Cholesterol/Tween 80/TPGS=25:45:20:5:3
(3) DOTAP/Egg PC/Cholesterol/Tween 80/TPGS=25:45:20:5:5

| Preparation | Particle size (nm) | Potential (mV) | Encapsulation rate (%) |
|---|---|---|---|
| 1 | 129.2 | 8.53 | 82.6% |
| 2 | 124.6 | 7.91 | 79.3% |
| 3 | 123.4 | 7.45 | 89.2% |

A single-factor experiment was performed by adjusting the molar ratio of TPGS, we found that under the condition that other components were always the same, the particle size and encapsulation rate of the third group were significantly superior than those of the other two groups, the potential remained substantially the same, and therefore we selected the third group as the optimal prescription for the following screening.
(1) DOTMA/Egg PC/Cholesterol/Tween 80/TPGS=25:45:20:5:5
(2) DOTMA/Egg PC/Cholesterol/Tween 80/TPGS=30:45:20:5:5
(3) DOTMA/Egg PC/Cholesterol/Tween 80/TPGS=35:45:20:5:5

| Preparation | Particle size (nm) | Potential (mV) | Encapsulation rate (%) |
|---|---|---|---|
| 1 | 131.4 | 5.76 | 88.4% |
| 2 | 127.2 | 6.66 | 91.9% |
| 3 | 126.4 | 6.75 | 84.6% |

In addition, we also screened for other potential phospholipids, a single-factor experiment was performed by adjusting the molar ratio of DOTMA positive phospholipids, we found that under the condition that other components were always the same, the particle size and encapsulation rate of the second group were significantly superior than those of the other two groups, the potential remained substantially the same, and therefore we selected the second group as the optimal prescription for the following screening.
(1) DOTAP/DSPC/Cholesterol/Tween 80/TPGS=30:40:20:5:5
(2) DOTAP/DSPC/Cholesterol/Tween 80/TPGS=30:45:20:5:5
(3) DOTAP/DSPC/Cholesterol/Tween 80/TPGS=30:50:20:5:5

| Preparation | Particle size (nm) | Potential (mV) | Encapsulation rate (%) |
|---|---|---|---|
| 1 | 124.4 | 7.12 | 82.8% |
| 2 | 126.0 | 8.04 | 87.1% |
| 3 | 131.8 | 7.71 | 89.9% |

A single-factor experiment was performed by adjusting the molar ratio of DSPC phospholipids, we found that under the condition that other components were always the same, the particle size and encapsulation rate of the third group were significantly superior than those of the other two groups, the potential remained substantially the same, and therefore we selected the third group as the optimal prescription for the following screening.
(1) DOTAP/Egg PC/Cholesterol/Tween 80/DPPE-mPEG2000=30:45:20:5:1
(2) DOTAP/Egg PC/Cholesterol/Tween 80/DPPE-mPEG2000=30:45:20:5:3

(3) DOTAP/Egg PC/Cholesterol/Tween 80/DPPE-mPEG2000=30:45:20:5:5

| Preparation | Particle size (nm) | Potential (mV) | Encapsulation rate (%) |
|---|---|---|---|
| 1 | 142.6 | 7.68 | 85.2% |
| 2 | 134.5 | 6.63 | 87.3% |
| 3 | 135.8 | 7.90 | 91.4% |

A single-factor experiment was performed by adjusting the molar ratio of DPPE-mPEG2000, we found that under the condition that other components were always the same, the particle size and encapsulation rate of the third group were significantly superior than those of the other two groups, the potential remained substantially the same, and therefore we selected the third group as the optimal prescription for the following screening.

After the composition of the above prescriptions and the screening of proportions, we can conclude that (1) DOTAP/Egg PC/Cholesterol/Tween 80/TPGS=25:45:20:5:5, the composition and ratio have smaller and stable particle size and encapsulation rate, and the positive potential is also relatively suitable. In addition, DOTMA/Egg PC/Cholesterol/Tween 80/TPGS=30:45:20:5:5, DOTAP/DSPC/Cholesterol/Tween 80/TPGS=30:50:20:5:5, DOTAP/Egg PC/ Cholesterol/Tween 80/-mPEG 2000-DPPE=30:45:20:5:5 are also preferred ratios among particle sizes, potentials, encapsulation rates, and stability in other component compositions. We will conduct further analysis experiments with these ratios and prescription compositions.

The following experiments were conducted for Example 1: free 2'-O-Me modified G3139 and 2'-O-Me modified G3139 lipid nanoparticles in the table represent G3139 (5'-UCU CCC AGC GTG CGC CAU-3') conducting phosphorothioate modification to the entire chain and 2'-O-Me modification to the 3 nucleotides on each end;

Free G3139 and G3139 lipid nanoparticles represent G3139 (5'-TCT CCC AGC GTG CGC CAT-3') conducting phosphorothioate modification to the entire chain;

The blank nanoparticles indicate that the non-content lipid nanoparticles were encapsulated by the membrane material.

1. A549 lung cancer cytotoxicity test

(1) We placed A549 cells in 96-well plates, and made the cells grow in an RPMI1640 culture solution containing 10% fetal calf serum to allow the cells to grow overnight to achieve a plating rate of 60-70%.
(2) A toxicity test of multiple paclitaxel (PTX) concentration gradients (1 nmol/L-100 μmol/L) and antisense oligonucleotide concentrations (10 μmol/L) in each group was conducted with each concentration group on the second day, and each concentration group was incubated in a 37 degree and 5% CO2 environment.
(3) 20 ul of MTS reagent (a novel tetrazole compound) was added at different time points 24 h, 48 h, and 72 h, and the detection was performed at a wavelength of 490 nm and the results were as shown in the following table:

| Therapeutic effect groups | IC50 (nmol/L) after 24 h | IC50 (nmol/L) after 48 h | IC50 (nmol/L) after 72 h |
|---|---|---|---|
| Paclitaxel (PTX) alone | 75.6 | 28.5 | 17.85 |
| PTX+free 2'-O-Me modified G3139 | 68.4 | 19.4 | 10.73 |
| PTX+free G3139 | 65.6 | 17.3 | 13.5 |
| PTX+blank nanoparticles | 88.4 | 31.5 | 15.9 |
| PTX+G3139 lipid nanoparticles | 57.6 | 11.5** | 9.9 |
| PTX+2'-O-Me modified | 73.5 | 8.53*** | 7.11** |
| G3139 lipid nanoparticles | | | |
| ** represents p<0.01, ***< represents p<0.001 | | | |

In this experiment, each experimental group was treated with different concentrations of paclitaxel (PTX), and the concentration of different antisense oligonucleotide groups was fixed to be 10umol/L to show its chemical sensitization.

We found that 48 h after the treatment of cancer cells, G3139 lipid nanoparticles and 2'-O-Me modified G3139 lipid nanoparticles can have chemical sensitization effect on the cancer cells to a certain extent, if it is compatible with paclitaxel for the treatment, the killing effect on cancer cells can be improved to a large extent, and there was statistically significant.

2. Gene level (Bcl-2) G3139 regulation experiment

By designing different experimental sample groups, we determined the regulatory effect of various G3139 dosage forms on the bcl-2 levels of a series of different cancer cells (including A549 cancer cells, KB cancer cells, and LNCap prostate cancer cells), and β-actin as an internal reference control gene, does not change with the drug treatment, bcl-2 is our target gene, and whether the drug works can be determined by observing its up and down regulations.

(1) We will place cancer cells, such as A549 cells, KB cells, and LNCaP prostate cancer cells in 6-well plates and make the cells grow in an RPMI1640 culture solution containing 10% fetal calf serum to allow the cells to grow overnight to achieve a plating rate of 80%.

(2) G3139 concentration preparation having 1 μM of final concentration was added to a tumor cell culture solution, and the preparation was divided into the following six groups, namely 2'-O-Me modified G3139 lipid nanoparticles, G3139 lipid nanoparticles, free G3139, free 2'-O-Me modified G3139, blank lipid nanoparticles, and culture solution control group.

(3) After incubation in an environment of 37 degrees and 5% CO2 for 4 h, the cells will be transferred to a fresh culture solution and the culture will last for 48 h. Cells were collected, lysed, and extracted RNA according to standard methods.

(4) We will analyze the levels of bcl-2 messenger RNA in each group with the Real-Time-PCR technology, and we can finally quantify the expression of bcl-2 after treatment in each group according to the standard methods of reagent suppliers.

1) KB cancer cells

By designing different sample groups, we regulate the levels of bcl-2 in KB cells. The test results are as shown in the following table and FIG. 2, β-actin is an internal reference control gene and does not change with the treatment, bcl-2 is our target gene, and whether the drug works can be determined by target gene regulations.

| Sequence numbers | Therapeutic effect groups | Ratio (bcl-2/β-actin) | Standard deviation |
|---|---|---|---|
| 1 | 2'-O-Me modified G3139 lipid nanoparticles | 32.29% | 4.14% |
| 2 | G3139 lipid nanoparticles | 56.28% | 8.08% |
| 3 | free G3139 | 95.31% | 5.11% |
| 4 | free 2'-O-Me modified G3139 | 89.04% | 5.66% |
| 5 | blank lipid nanoparticles | 135.28% | 9.11% |
| 6 | control group | 100.00% | 4.10% |

100.00% 4.10% From the above table and FIG. 2, the 2'-O-Me modified lipid nanoparticles have a 67.71% gene-level down-regulation, as compared with other experimental groups, the bcl-2 level has a large degree of down-regulation, while the free drug group (3,4) cannot enter the cells due to the lack of a drug delivery system, but only has a weak downregulation (4.69% and 10.96%), and blank nanoparticles cannot play a role of down-regulation because there is no drug.

2) A549 lung cancer cells

The test results are as shown in the following table and FIG. 3:

| | Ratio (bcl-2/β-actin) | Standard deviation |
|---|---|---|
| 2'-O-Me modified G3139 lipid nanoparticles | 28.13% | 5.46% |
| G3139 lipid nanoparticles | 37.65% | 7.27% |
| free G3139 | 88.75% | 6.11% |
| free 2'-O-Me modified G3139 | 93.29% | 5.93% |
| blank lipid nanoparticles | 121.67% | 10.81% |
| control group | 100% | 5.09% |

From the above table and FIG. 3, the 2'-O-Me modified lipid nanoparticles have a 71.87% gene-level down-regulation, as compared with other experimental groups, the bcl-2 level has a large degree of down-regulation, while the free drug group (3,4) cannot enter the cells due to the lack of a drug delivery system, but only has a weak downregulation (11.25% and 6.71%), and blank nanoparticles cannot play a role of down-regulation because there is no drug.

3) LNCaP prostate cancer cells

The test results are as shown in the following table and FIG. 4:

| | Ratio (bcl-2/β-actin) | Standard deviation |
|---|---|---|
| 2'-O-Me modified G3139 lipid nanoparticles | 21.22% | 3.68% |
| G3139 lipid nanoparticles | 50.92% | 7.27% |
| free G3139 | 93.15% | 4.46% |
| free 2'-O-Me modified G3139 | 94.89% | 3.95% |
| blank lipid nanoparticles | 144.73% | 8.33% |
| control group | 100% | 4.31% |

100%4.31% From the above table and FIG. 4, the 2'-O-Me modified lipid nanoparticles have a 78.78% gene-level down-regulation, as compared with other experimental groups, the bcl-2 level has a large degree of down-regulation, while the free drug group (3,4) cannot enter the cells due to the lack of a drug delivery system, but only has a weak downregulation (6.85% and 5.11%), and blank nanoparticles cannot play a role of down-regulation because there is no drug.

3. Pharmacodynamics of ectopically inoculating KB cells into tumor cell models in mice and survival curve test

(1) $5*10^6$ KB cancer cells will be inoculated into the right back of nude mice and allowed to grow for 1-2 weeks to a visible tumor of an average 150 $mm^3$.

(2) When the tumors reach the expected size, each group of tumor-bearing mice will be given different types of drugs through the caudal vein, namely 2'-O-Me modified G3139 lipid nanoparticles, G3139 lipid nanoparticles, free G3139, free 2'-O-Me modified G3139, paclitaxel injection, and normal saline. Drugs are given once every three days for three weeks. The treated groups are divided into 6 groups with 10 tumor-bearing mice in each group.

(3) The size including length and width of the tumor will be measured by the Vernier caliper, and volume of the tumor is given by the following formula:

$$\text{Tumor volume}=(\pi/6)*\text{length (mm)}*(\text{width})^2$$

(4) When the actual tumor size reaches 1500 $mm^3$, the mice will be removed from the treated group and euthanized through carbon dioxide asphyxiation.

The volume of tumor in each experimental group after the end of the treatment cycle is an indication of antitumor activity.

FIG. 5 and FIG. 6 are graphs of the tumor inhibition rate test and the survival test.

From FIG. 5 and FIG. 6, and from the perspective of the pharmacodynamics of xenografts of KB cells in nude mice and the survival curve, we can see that the 2'-O-Me modified G3139 lipid nanoparticles are combined with paclitaxel to exert fairly good tumor inhibiting effect and long survival time of tumor-bearing mice, which indicates that the lipid nanoparticles do not produce significant toxic side effects while improving the efficacy of the drug.

## Claims

1. A lipid nanoparticle comprising antisense oligonucleotides for inhibiting bcl-2, wherein the lipid nanoparticle is prepared by coating an antisense oligonucleotide with a membrane material, and

   the nucleic acid sequence of the antisense oligonucleotide is 5'-TCT CCC AGC GTG CGC CAT-3', or 5' UCU CCC AGC GTG CGC CAU 3'; and
   the membrane material comprises a cationic lipid, a neutral phospholipid, cholesterol, Tween ®, a polyethylene glycol derivative in a molar ratio of (25-35):(40-50):(15-25):(1-5):(1-5);
   wherein the PEG in the polyethylene glycol derivative includes monomethoxy polyethylene glycol having a molecular weight of from 550 to 5,000; and
   the Tween ® is Tween ® 80.

2. The lipid nanoparticle comprising antisense oligonucleotides for inhibiting bcl-2 according to claim 1, wherein: the cationic lipid includes: DOTAP, DOTMA, DDAB, and DODMA;

   the neutral phospholipid includes: egg PC, DOPC, DSPC, DPPC, and DMPC; and
   the polyethylene glycol derivative includes: mPEG-DPPE, mPEG-DMPE, mPEG-DSPE, TPGS, and mPEG-cholesterol.

3. The lipid nanoparticle comprising antisense oligonucleotides for inhibiting bcl-2 according to claim 2, wherein the polyethylene glycol derivative is a substance formed by linking a hydrophilic polyethylene glycol chain or a methoxy polyethylene glycol chain to a hydrophobic structure that can be embedded into a phospholipid bilayer; the hydrophobic structure includes cholesterol, vitamin E, dipalmitoylphosphatidylethanolamine, dimyristoylphosphatidylethanolamine, dimyristoylglycerol or structural analogs.

4. The lipid nanoparticle comprising antisense oligonucleotides for inhibiting bcl-2 according to claim 1, wherein a modification to the antisense oligonucleotide 5' UCU CCC AGC GTG CGC CAU 3' is a 2'-O-Me modification to 3 nucleotides at both ends thereof, and a phosphorothioate modification to an entire chain; or phosphorothioate modification to an entire chain of antisense oligonucleotide 5'-TCT CCC AGC GTG CGC CAT-3'.

5. The lipid nanoparticle comprising antisense oligonucleotides for inhibiting bcl-2 according to claim 2, wherein the membrane material comprises DOTAP, Egg PC, Cholesterol, Tween ® 80, and TPGS in a molar ratio of 25:45:20:5:5.

6. The lipid nanoparticle comprising antisense oligonucleotides for inhibiting bcl-2 according to claim 2, wherein the membrane material comprises DOTMA, Egg PC, Cholesterol, Tween ® 80, and TPGS in a molar ratio of 30:45:20:5:5.

7. The lipid nanoparticle comprising antisense oligonucleotides for inhibiting bcl-2 according to claim 2, wherein the membrane material comprises DOTAP, DSPC, Cholesterol, Tween ® 80, and TPGS in a molar ratio of 30:50:20:5:5.

8. The lipid nanoparticle comprising antisense oligonucleotides for inhibiting bcl-2 according to claim 2, wherein the membrane material comprises DOTAP, Egg PC, Cholesterol, Tween ® 80, and mPEG2000-DPPE in a molar ratio of 30:45:20:5:5.

## Patentansprüche

1. Lipid-Nanopartikel umfassend Antisense-Oligonukleotide zur Hemmung von bcl-2, wobei der

Lipid-Nanopartikel durch Beschichten eines Antisense-Oligonukleotids mit einem Membranmaterial hergestellt wird und die Nukleinsäuresequenz des Antisense-Oligonukleotids 5'-TCT CCC AGC GTG CGC CAT-3' oder 5' UCU CCC AGC GTG CGC CAU 3' ist; und
wobei das Membranmaterial ein kationisches Lipid, ein neutrales Phospholipid, Cholesterin, Tween ®, ein Polyethylenglykolderivat in einem molaren Verhältnis von (25-35):(40-50):(15-25):(1-5):(1-5) umfasst;
wobei das PEG in dem Polyethylenglykolderivat Monomethoxypolyethylenglykol mit einem Molekulargewicht von 550 bis 5000 beinhaltet; und
das Tween ® ist Tween ® 80.

2. Lipid-Nanopartikel umfassend Antisense-Oligonukleotide zur Hemmung von bcl-2 nach Anspruch 1, wobei: das kationische Lipid umfasst: DOTAP, DOTMA, DDAB, und DODMA;

   woei das neutrale Phospholipid umfasst: Ei-PC, DOPC, DSPC, DPPC und DMPC; und
   wobei das Polyethylenglykolderivat umfasst: mPEG-DPPE, mPEG-DMPE, mPEG-DSPE, TPGS und mPEG-Cholesterin.

3. Lipid-Nanopartikel umfassend Antisense-Oligonukleotide zur Hemmung von bcl-2 nach Anspruch 2, wobei das Polyethylenglykolderivat eine Substanz ist, die durch Binden einer hydrophilen Polyethylenglykolkette oder einer Methoxy-Polyethylenglykolkette an eine hydrophobe Struktur gebildet wird, die in eine Phospholipid-Doppelschicht eingebettet werden kann; wobei die hydrophobe Struktur umfasst: Cholesterin, Vitamin E, Dipalmitoylphosphatidylethanolamin, Dimyristoylphosphatidylethanolamin, Dimyristoylglycerin oder strukturelle Analoga.

4. Lipid-Nanopartikel umfassend Antisense-Oligonukleotide zur Hemmung von bcl-2 nach Anspruch 1, wobei eine Modifikation des Antisense-Oligonukleotids 5' UCU CCC AGC GTG CGC CAU 3' eine 2'-O-Me-Modifikation an 3 Nukleotiden an beiden Enden davon ist, und eine Phosphorothioat-Modifikation an einer gesamten Kette ist; oder eine Phosphorothioat-Modifikation an einer gesamten Kette des Antisense-Oligonukleotids 5'-TCT CCC AGC GTG CGC CAT-3' ist.

5. Lipid-Nanopartikel umfassend Antisense-Oligonukleotide zur Hemmung von bcl-2 nach Anspruch 2, wobei das Membranmaterial DOTAP, Ei-PC, Cholesterin, Tween ® 80 und TPGS in einem molaren Verhältnis von 25:45:20:5:5 umfasst.

6. Lipid-Nanopartikel umfassend Antisense-Oligonukleotide zur Hemmung von bcl-2 nach Anspruch 2, wobei das Membranmaterial DOTMA, Ei-PC, Cholesterol, Tween ® 80 und TPGS in einem Molverhältnis von 30:45:20:5:5 umfasst.

7. Lipid-Nanopartikel umfassend Antisense-Oligonukleotide zur Hemmung von bcl-2 nach Anspruch 2, wobei das Membranmaterial DOTAP, DSPC, Cholesterin, Tween ® 80 und TPGS in einem Molverhältnis von 30:50:20:5:5 umfasst.

8. Lipid-Nanopartikel umfassend Antisense-Oligonukleotide zur Hemmung von bcl-2 nach Anspruch 2, wobei das Membranmaterial DOTAP, Ei-PC, Cholesterol, Tween ® 80 und mPEG2000-DPPE in einem Molverhältnis von 30:45:20:5:5 umfasst.

## Revendications

1. Une nanoparticule lipidique comprenant des oligonucléotides antisens pour inhiber bcl-2, dans

   laquelle la nanoparticule lipidique étant préparée en enrobant un oligonucléotide antisens avec un matériau membranaire, et la séquence d'acide nucléique de l'oligonucléotide antisens est 5'-TCT CCC AGC GTG CGC CAT-3' , ou 5' UCU CCC AGC GTG CGC CAU 3' ; et
   le matériau membranaire comprend un lipide cationique, un phospholipide neutre, du cholestérol, du Tween ®, un dérivé de polyéthylène glycol dans un rapport molaire de (25-35):(40-50):(15-25):(1-5) : (1-5);
   dans lequel le PEG dans le dérivé de polyéthylène glycol comprend du monométhoxy polyéthylène glycol ayant un poids moléculaire de 550 à 5 000 ; et
   le Tween ® est le Tween ® 80.

2. La nanoparticule lipidique comprenant des oligonucléotides antisens pour inhiber bcl-2 selon la revendication 1, dans laquelle : le lipide cationique comprend : DOTAP, DOTMA, DDAB, et DODMA ;

le phospholipide neutre comprend : PC d'oeuf, DOPC, DSPC, DPPC, et DMPC ; et
le dérivé de polyéthylène glycol comprend : mPEG-DPPE, mPEG-DMPE, mPEG-DSPE, TPGS, et mPEG-cholestérol.

3. La nanoparticule lipidique comprenant des oligonucléotides antisens pour inhiber bcl-2 selon la revendication 2, dans laquelle le dérivé de polyéthylène glycol est une substance formée en liant une chaîne de polyéthylène glycol hydrophile ou une chaîne de polyéthylène glycol méthoxy à une structure hydrophobe qui peut être intégrée dans une bicouche phospholipidique ; la structure hydrophobe comprend le cholestérol, la vitamine E, la dipalmitoylphosphatidyléthanolamine, la dimyristoylphosphatidyléthanolamine, le dimyristoylglycérol ou des analogues structuraux.

4. La nanoparticule lipidique comprenant des oligonucléotides antisens pour inhiber bcl-2 selon la revendication 1, dans laquelle une modification de l'oligonucléotide antisens 5' UCU CCC AGC GTG CGC CAU 3' est une modification 2'-O-Me de 3 nucléotides à ses deux extrémités, et une modification phosphorothioate sur une chaîne entière ; ou une modification phosphorothioate d'une chaîne entière d'oligonucléotide antisens 5'-TCT CCC AGC GTG CGC CAT-3'.

5. La nanoparticule lipidique comprenant des oligonucléotides antisens pour inhiber bcl-2 selon la revendication 2, dans laquelle le matériau membranaire comprend du DOTAP, PC d'oeuf, Cholesterol, Tween ® 80, et TPGS dans un rapport molaire de 25:45:20:5:5.

6. La nanoparticule lipidique comprenant des oligonucléotides antisens pour inhiber bcl-2 selon la revendication 2, dans laquelle le matériau membranaire comprend du DOTMA, Egg PC, Cholesterol, Tween ® 80, et TPGS dans un rapport molaire de 30:45:20:5:5.

7. La nanoparticule lipidique comprenant des oligonucléotides antisens pour inhiber bcl-2 selon la revendication 2, dans laquelle le matériau membranaire comprend du DOTAP, DSPC, Cholesterol, Tween ® 80, et TPGS dans un rapport molaire de 30:50:20:5:5.

8. La nanoparticule lipidique comprenant des oligonucléotides antisens pour inhiber bcl-2 selon la revendication 2, dans laquelle le matériau membranaire comprend du DOTAP, Egg PC, Cholesterol, Tween ® 80, et mPEG2000-DPPE dans un rapport molaire de 30:45:20:5:5.

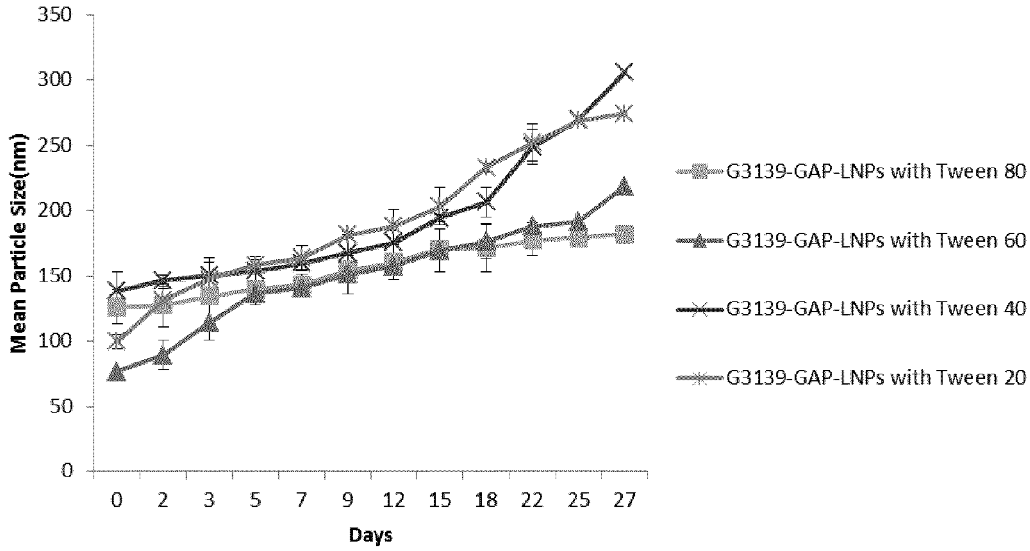

Mean Particle Size(nm)
350
300
250
200
150
100
50
0
0 2 3 5 7 9 12 15 18 22 25 27
Days
G3139-GAP-LNPs with Tween 80
G3139-GAP-LNPs with Tween 60
G3139-GAP-LNPs with Tween 40
G3139-GAP-LNPs with Tween 20


FIG. 1

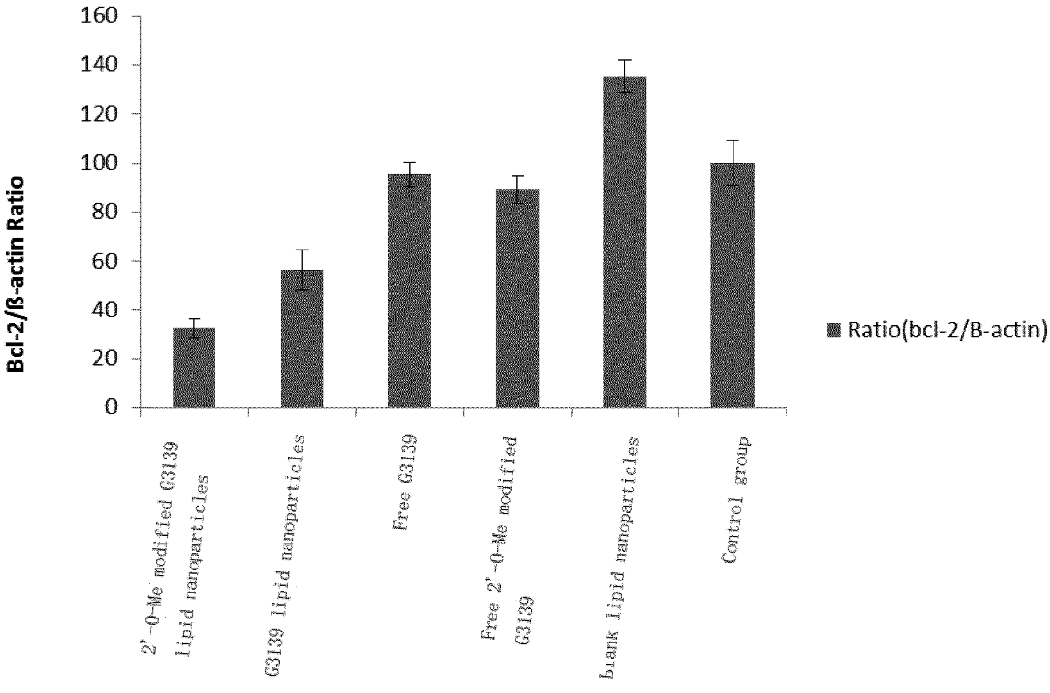

Bcl-2/ß-actin Ratio
160
140
120
100
80
60
40
20
0
2'-O-Me modified G3139 lipid nanoparticles
G3139 lipid nanoparticles
Free G3139
Free 2'-O-Me modified G3139
blank lipid nanoparticles
Control group
Ratio(bcl-2/B-actin)


FIG. 2

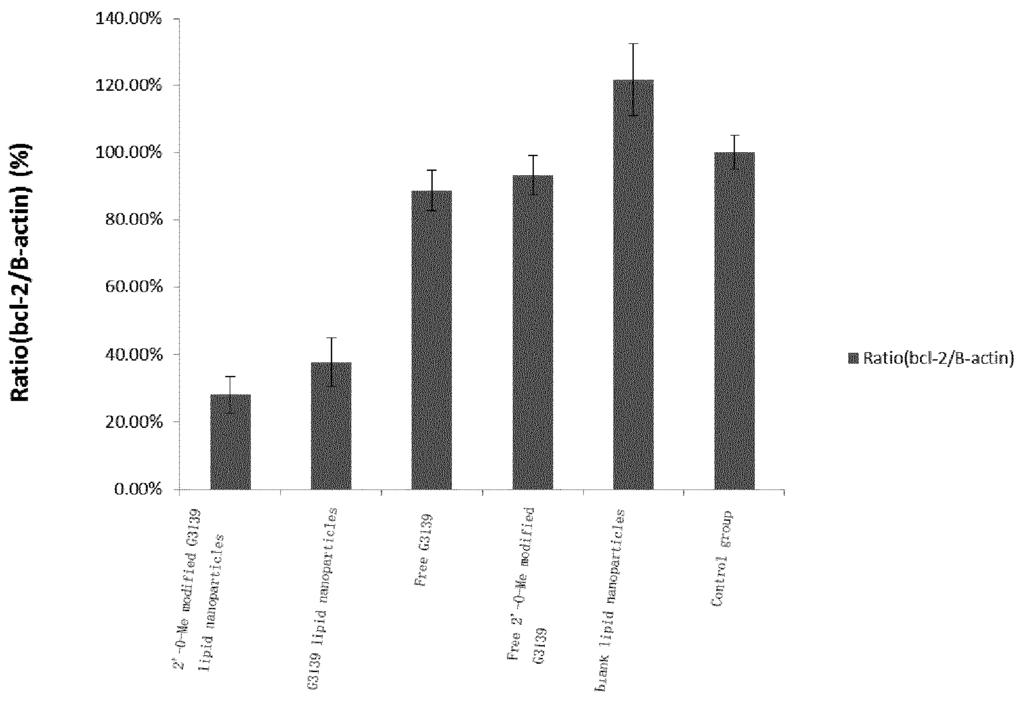
Ratio(bcl-2/B-actin) (%)
140.00%
120.00%
100.00%
80.00%
60.00%
40.00%
20.00%
0.00%
2'-O-Me modified G3139 lipid nanoparticles
G3139 lipid nanoparticles
Free G3139
Free 2'-O-Me modified G3139
blank lipid nanoparticles
Control group
Ratio(bcl-2/B-actin)

FIG. 3

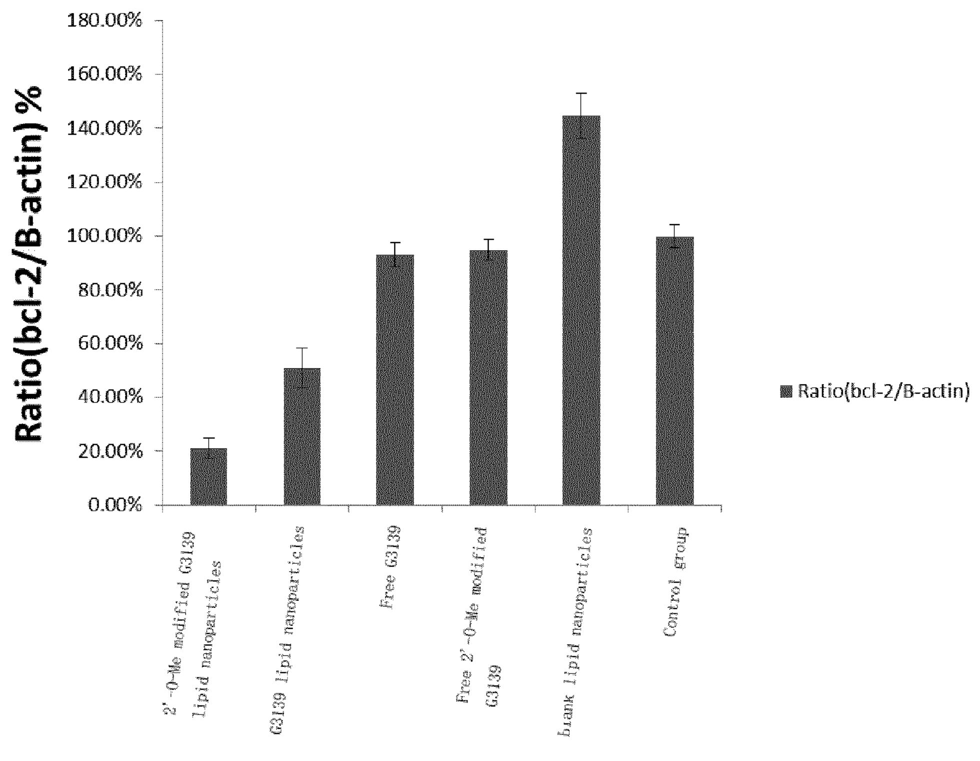
Ratio(bcl-2/B-actin) %
180.00%
160.00%
140.00%
120.00%
100.00%
80.00%
60.00%
40.00%
20.00%
0.00%
2'-O-Me modified G3139 lipid nanoparticles
G3139 lipid nanoparticles
Free G3139
Free 2'-O-Me modified G3139
blank lipid nanoparticles
Control group
Ratio(bcl-2/B-actin)

FIG. 4

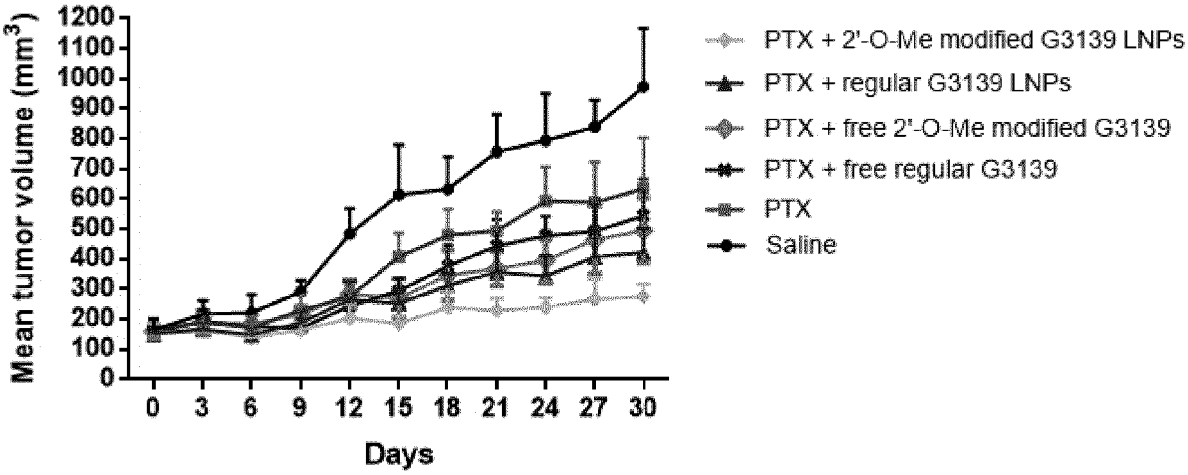
Mean tumor volume (mm3)
1200
1100
1000
900
800
700
600
500
400
300
200
100
0
0
3
6
9
12
15
18
21
24
27
30
Days
PTX + 2'-O-Me modified G3139 LNPs
PTX + regular G3139 LNPs
PTX + free 2'-O-Me modified G3139
PTX + free regular G3139
PTX
Saline

FIG. 5

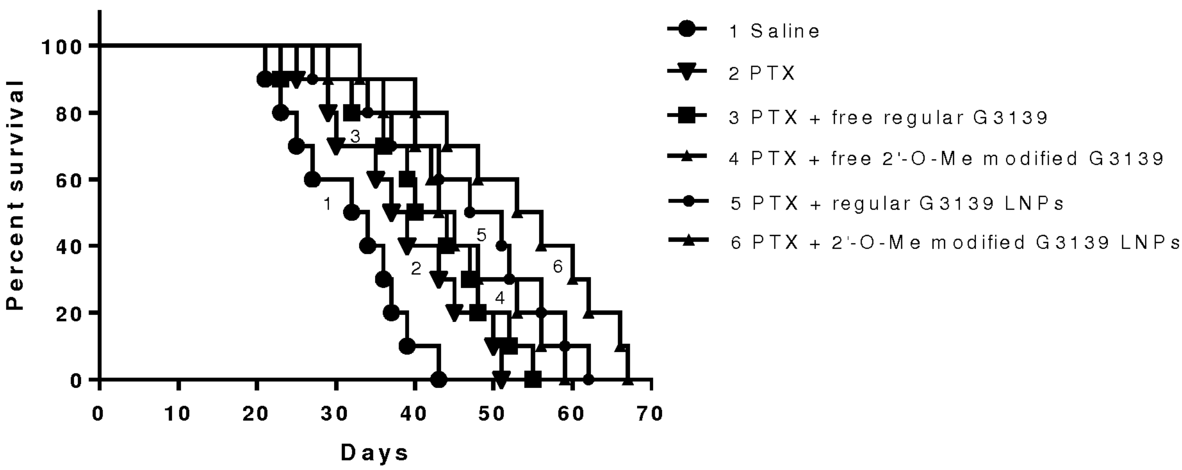
Percent survival
100
80
60
40
20
0
0
10
20
30
40
50
60
70
Days
1 Saline
2 PTX
3 PTX + free regular G3139
4 PTX + free 2'-O-Me modified G3139
5 PTX + regular G3139 LNPs
6 PTX + 2'-O-Me modified G3139 LNPs

FIG. 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2009120247 A2